# EUROPEAN PATENT APPLICATION

(11) **EP 4 502 616 A1**
(43) Date of publication of application: **05.02.2025**
(21) Application number: 23907384.4
(22) Date of filing: 02.11.2023
(51) Int. Cl.: G01N 35/00, G01N 33/00

(54) **GAS ANALYSIS DEVICE**

(30) Priority: 20.12.2022 KR 20220178983
(71) Applicant: LG Energy Solution, Ltd., Seoul 07335 (KR)
(72) Inventor: JUNG, Ji Young, Daejeon 34122 (KR); CHOI, Nak Hee, Daejeon 34122 (KR); PARK, Kwangyeon, Daejeon 34122 (KR)
(74) Representative: Goddar, Heinz J.
(86) International application number: PCT/KR2023/017339
(87) International publication number: WO 2024/136098

(57) **Abstract**

The present invention relates to a gas analysis device, and is to provide a gas analysis device in which individually gases generated from a plurality of batteries to be analyzed can be collected and analyzed in an automated system.

## Description

### Technical Field

The present application claims the benefit of priority based on Korean Patent Application No. 10-2022-0178983 filed on December 20, 2022, the entire disclosure of which is incorporated as a part of this specification.

The present disclosure relates to a gas analysis apparatus, and to a gas analysis apparatus capable of individually collecting and analyzing gases generated from each of a plurality of batteries to be analyzed using an automated system.

### Background Art

In general, a secondary battery is a battery which can be used repeatedly through a discharging process in which chemical energy is converted into electrical energy and a charging process in the opposite direction to the discharging process and which includes nickelcadmium (Ni-Cd) batteries, nickel-metal hydride (Ni-MH) batteries, lithium-metal batteries, lithium-ion (Li-ion) batteries, and lithium-ion polymer batteries. Among these secondary batteries, lithium secondary batteries having high energy density and voltage, long cycle life, and a low self-discharge rate have been commercialized and widely used.

Depending on the reaction within the lithium secondary battery, various types of gases such as hydrogen, oxygen, nitrogen, carbon monoxide, carbon dioxide, hydrocarbons of CₙH₂ₙ₋₂ (n=2~5), CₙH₂ₙ (n=2~5), and CₙH₂ₙ₊₂ (n=1~5), and other organic gas species, may be generated as secondary battery generated gas.

In addition, the lithium secondary battery degrades while generating a large amount of secondary battery generated gas due to electrolyte decomposition as repeated charging and discharging progresses, and this aspect appears differently depending on the design and use form of the battery. Thus, it is essential to infer a deterioration mechanism of a battery by analyzing the secondary battery generated gas during a battery development process.

Therefore, it is very important to accurately analyze the secondary battery generated gas. Specifically, information on composition and content of the secondary battery generated gas is useful for developing battery materials, optimizing battery manufacturing processes, and identifying causes of battery defects. To this end, it is important to develop a technology to analyze the secondary battery generated gas.

Analysis of the secondary battery generated gas may be performed by steps of collecting gas generated inside the secondary battery, delivering the collected gas to an analysis apparatus such as a gas chromatography (GC) for GC measurement, and analyzing measurement data. Since the composition and amount of the secondary battery generated gas varies depending on not only the type of secondary battery, but also the conditions given to the secondary battery such as cycle number, SOC, and temperature, individual analysis is necessary for various types of secondary batteries, and individual collection and analysis of secondary battery generated gas is necessary for each of the various conditions. In other words, analysis of secondary battery generated gas may be repeatedly performed on a plurality of secondary batteries.

This repetitive work increases fatigue of an analyst as the number of samples increases, and human error may also occur depending on the skill level of the analyst. In addition, secondary battery analysis may be performed for a long time, and in this case, there were limitations to the analysis method performed by manual operations of the analyst.

Therefore, there is a need for automated analysis technology capable of automating gas collection in secondary batteries and GC measurement operations of the collected gas to minimize human errors, and enable efficient and accurate analysis.

### Disclosure of the Invention

Technical Goals

The present disclosure relates to a gas analysis apparatus, and is to provide a gas analysis apparatus capable of individually collecting and analyzing gases generated from each of a plurality of batteries to be analyzed using an automated system.

Technical objects to be achieved by the present disclosure are not limited to the technical objects mentioned above, and other technical objects not mentioned will be clearly understood by those skilled in the art from the description below.

### Technical Solutions

A gas analysis apparatus of the present disclosure may include:
a jig part on which a battery to be analyzed is mounted;
a gas diffusion chamber provided with a gas diffusion space in which the jig part is accommodated inside;
a supply battery storage unit provided with a supply battery storage space in which the jig part for supplying to the gas diffusion space is stored;
a recovery battery storage unit provided with a recovery battery storage space in which the jig part recovered from the gas diffusion space is stored;
a battery moving unit configured to move the jig part from the battery supply part to the gas diffusion space or move the jig part accommodated in the gas diffusion space to the battery recovery part;
a gas analysis unit configured to analyze gas delivered from the gas diffusion space; and
a gas delivery flow path configured to connect the gas diffusion space and the gas analysis unit.

### Advantageous Effects

A gas analysis apparatus of the present disclosure is an automated system which may be capable of collecting and analyzing secondary battery generated gas by minimizing intervention of the analyst.

The gas analysis apparatus of the present disclosure may be capable of independently collecting and analyzing gases for a plurality of secondary batteries with only initial settings of the analyst.

The gas analysis apparatus of the present disclosure may be capable of continuous analysis even if the analyst is not waiting near the gas analysis apparatus.

### Brief Description of Drawings

FIG. 1 is a perspective view illustrating a gas analysis apparatus of the present disclosure.
FIG. 2 is a perspective view illustrating a jig part.
FIG. 3 is a perspective view illustrating a gripper part.
FIG. 4 is a cross-sectional view illustrating a state in which the gripper part is gripping the jig part.
FIG. 5 is a perspective view illustrating a supply battery storage unit.
FIG. 6 is a perspective view illustrating a recovery battery storage unit.
FIG. 7 is a perspective view illustrating a gas diffusion chamber.

### Best Mode for Carrying Out the Invention

A gas analysis apparatus of the present disclosure may include:
a jig part on which a battery to be analyzed is mounted;
a gas diffusion chamber provided with a gas diffusion space in which the jig part is accommodated inside;
a supply battery storage unit provided with a supply battery storage space in which the jig part for supplying to the gas diffusion space is stored;
a recovery battery storage unit provided with a recovery battery storage space in which the jig part recovered from the gas diffusion space is stored;
a battery moving unit configured to move the jig part from the battery supply part to the gas diffusion space or move the jig part accommodated in the gas diffusion space to the battery recovery part;
a gas analysis unit configured to analyze gas delivered from the gas diffusion space; and
a gas delivery flow path configured to connect the gas diffusion space and the gas analysis unit.

In the gas analysis apparatus of the present disclosure, a battery accommodation groove for mounting the battery to be analyzed may be formed on an upper surface of the jig part.

In the gas analysis apparatus of the present disclosure, the battery moving unit may include a gripper part for gripping the jig part, an elevation driving part for linearly moving the gripper part in a vertical direction, and a first driving part for moving the gripper part in a first direction perpendicular to the vertical direction.

In the gas analysis apparatus of the present disclosure, when a direction perpendicular to the vertical direction and the first direction is referred to as a second direction, the gripper part may include a pair of grip members configured to grip the jig part by contacting each of both side surfaces of the jig part formed as a plane perpendicular to the second direction.

In the gas analysis apparatus of the present disclosure, a pair of gripper insertion groove parts may be formed in the jig part, and each of the pair of gripper insertion groove parts may be located on each of the both side surfaces of the jig part formed as the plane perpendicular to the second direction.

In the gas analysis apparatus of the present disclosure, each of the pair of gripper insertion groove parts may include a first gripper insertion groove formed in a long groove shape, and a second gripper insertion groove formed to be spaced a predetermined distance apart from the first gripper insertion groove.

In the gas analysis apparatus of the present disclosure, a first protrusion to be inserted into the first gripper insertion groove and a second protrusion to be inserted into the second gripper insertion groove may be formed on the pair of grip members.

In the gas analysis apparatus of the present disclosure, the gripper part may further include a grip actuator configured to provide driving force to adjust a gap between the pair of grip members, and a gripper body member to which the grip actuator and the pair of grip members are coupled, the first driving part may include a first moving member configured to move linearly in the first direction, and a first guide member configured to guide a movement of the first moving member, the elevation driving part may include an elevation guide member coupled to the first moving member, and an elevation moving member configured to move linearly in the vertical direction along the elevation guide member, and the gripper body member may be fixed to the elevation moving member.

In the gas analysis apparatus of the present disclosure, the supply battery storage unit may include a supply battery storage part provided with the supply battery storage space inside and extending in the vertical direction, and a supply battery lifting part configured to lift up the jig part in the supply battery storage space, a battery discharge port may be formed at an upper end of the supply battery storage part, and a first gripper contact hole may be formed on each of both side surfaces of the supply battery storage part so that the pair of grip members can directly contact each of the both side surfaces of the jig part formed as the plane perpendicular to the second direction.

In the gas analysis apparatus of the present disclosure, the recovery battery storage unit may include a recovery battery storage part provided with the recovery battery storage space inside and extending in the vertical direction, and a recovery battery lowering part configured to lower the jig part in the recovery battery storage space, a battery recovery port may be formed at an upper end of the recovery battery storage part, and a second gripper contact hole may be formed on each of both side surfaces of the recovery battery storage part so that the pair of grip members can directly contact each of the both side surfaces of the jig part formed as the plane perpendicular to the second direction.

In the gas analysis apparatus of the present disclosure, the gas diffusion chamber may be located to be spaced a predetermined distance apart from the supply battery storage unit and the recovery battery storage unit in the first direction, the gas diffusion chamber may include a chamber body part, wherein a jig part accommodation groove is formed on an upper surface of the chamber body part as the gas diffusion space, and a chamber cover part configured to cover the upper surface of the chamber body part to make the gas diffusion space a closed space, and the chamber cover part may be configured to open or close the gas diffusion space by sliding in a second direction perpendicular to the vertical direction and the first direction.

In the gas analysis apparatus of the present disclosure, a punching part for perforating the battery to be analyzed may be provided on an upper surface of the chamber cover part, and a punching needle of the punching part may be configured to perforate the battery to be analyzed by being inserted into the gas diffusion space through a penetration hole of the chamber cover part.

In the gas analysis apparatus of the present disclosure, the gas delivery flow path may be connected to the chamber body part.

In the gas analysis apparatus of the present disclosure, a manifold may be provided in the gas delivery flow path, and the gas delivered from the gas diffusion space to the gas analysis unit through the gas delivery flow path may be injected into the gas analysis unit after waiting in the manifold for a predetermined period of time.

### Modes for Carrying Out the Invention

Hereinafter, embodiments according to the present disclosure will be described in detail with reference to the accompanying drawings. In this process, the size or shape of the components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that an orientation or positional relationship indicated by the terms such as "center", "upper", "lower", "left", "right", "vertical", "horizontal", "inner side", "outer side", "one side", and "other side" is based on an orientation or positional relationship shown in a drawing or an orientation or positional relationship that is placed when using the product of the present disclosure on a daily basis, and is merely for explanation and brief description of the present disclosure, and it does not suggest or imply that the displayed device or element must necessarily be configured or operated in a specified orientation and should not be construed as limiting the present disclosure.

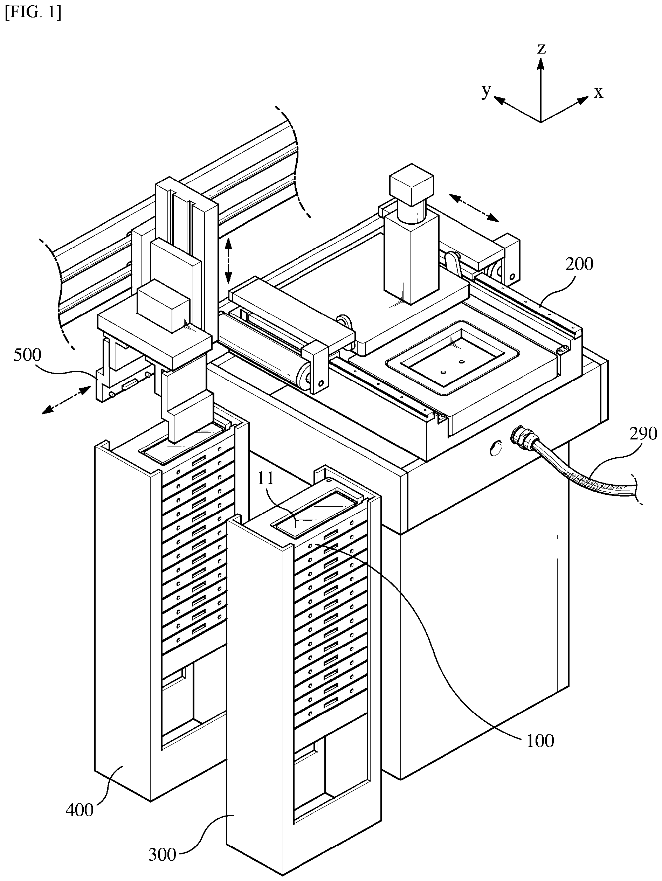

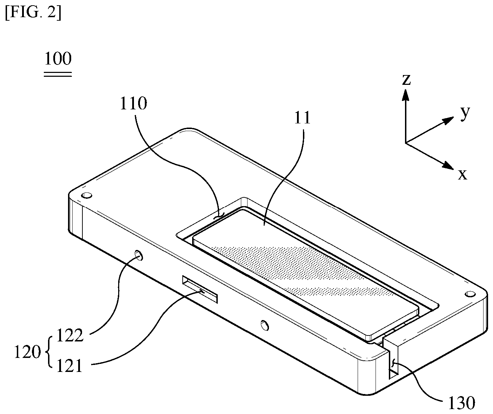

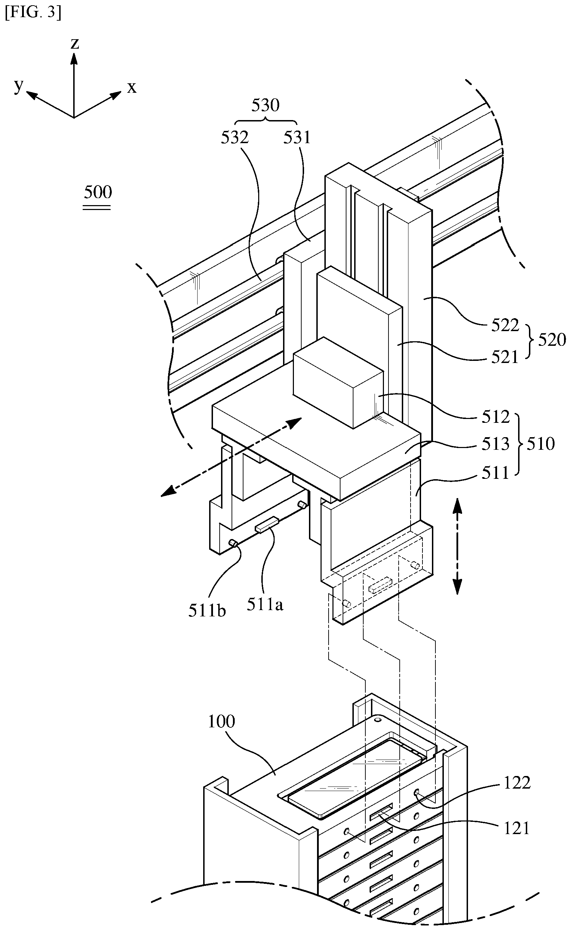

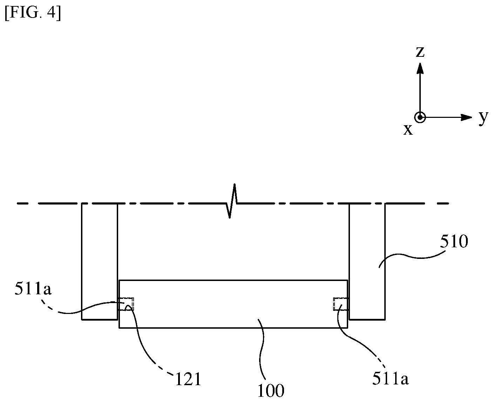

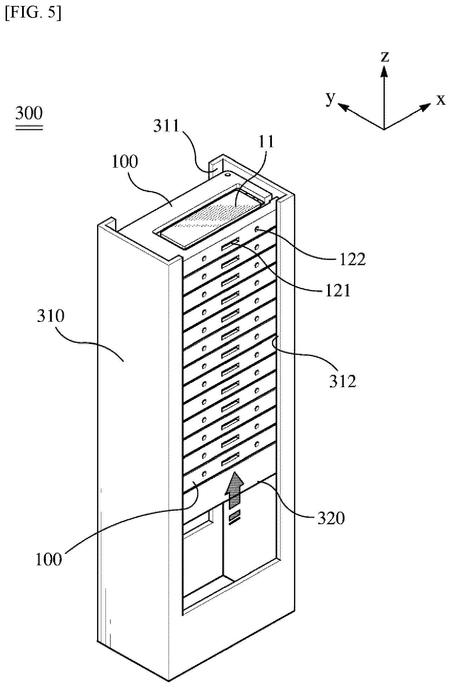

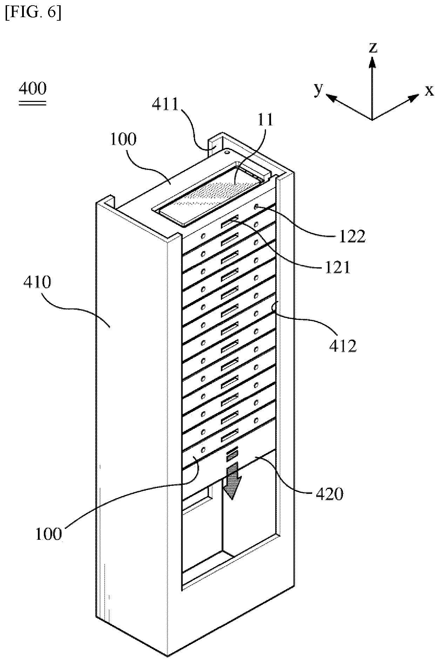

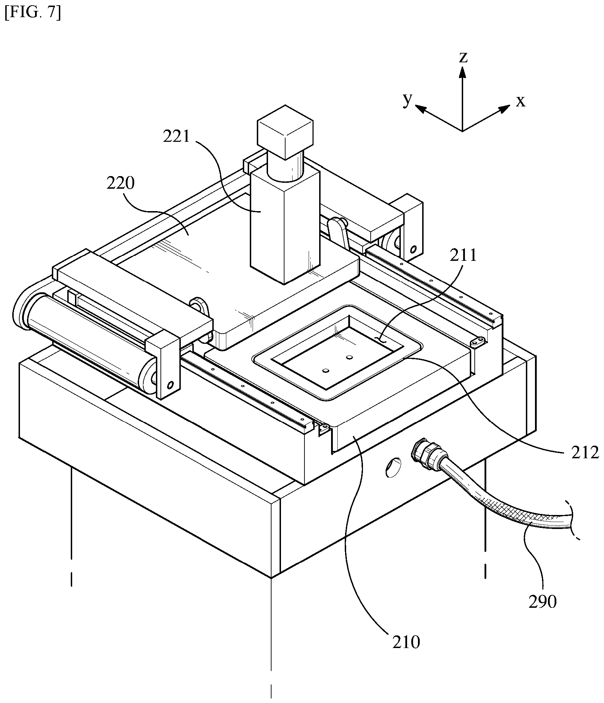

FIG. 1 is a perspective view illustrating a gas analysis apparatus of the present disclosure. FIG. 2 is a perspective view illustrating a jig part 100. FIG. 3 is a perspective view illustrating a gripper part 510. FIG. 4 is a cross-sectional view illustrating a state in which the gripper part 510 is gripping the jig part 100. FIG. 5 is a perspective view illustrating a supply battery storage unit 300. FIG. 6 is a perspective view illustrating a recovery battery storage unit 400. FIG. 7 is a perspective view illustrating a gas diffusion chamber 200.

Hereinafter, the gas analysis apparatus of the present disclosure will be described in detail with reference to FIGS. 1 to 7.

Hereinafter, in the coordinate system shown in FIGS. 1 to 7, the z-axis direction may be a vertical direction, the x-axis direction may be a first direction, and the y-axis direction may be a second direction.

The gas analysis apparatus of the present disclosure is for analyzing gas generated inside a secondary battery, and may be capable of loading a battery to be analyzed 11 into a closed space, diffusing the gas generated from the battery to be analyzed 11 into the closed space, collecting the diffused gas, delivering the collected gas to a gas analysis unit to perform gas analysis, and unloading the battery to be analyzed 11 after the analysis from the closed space, without manual work by the analyst.

Therefore, the gas analysis apparatus of the present disclosure may ensure the safety of the analyst and minimize human errors caused by manipulation of the analyst during the gas collection and analysis process.

As shown in FIG. 1, the gas analysis apparatus of the present disclosure may include:
the jig part 100 on which the battery to be analyzed 11 is mounted;
the gas diffusion chamber 200 provided with a gas diffusion space in which the jig part 100 is accommodated inside;
the supply battery storage unit 300 provided with a supply battery storage space in which the jig part 100 for supplying to the gas diffusion space is stored;
the recovery battery storage unit 400 provided with a recovery battery storage space in which the jig part 100 recovered from the gas diffusion space is stored;
a battery moving unit 500 configured to move the jig part 100 from the battery supply part to the gas diffusion space or move the jig part 100 accommodated in the gas diffusion space to the battery recovery part;
a gas analysis unit configured to analyze gas delivered from the gas diffusion space; and
a gas delivery flow path 290 configured to connect the gas diffusion space and the gas analysis unit.

The gas analysis unit may be equipment capable of quantitative and qualitative analysis of gas using gas chromatography (GC) analysis equipment, and the like. For example, the gas analysis unit may be gas chromatography/mass spectrometer (GC/MS) analysis equipment.

In the gas analysis apparatus of the present disclosure, the battery to be analyzed 11 may not be accommodated alone in the gas diffusion space provided inside the gas diffusion chamber 200, but may be mounted on the jig part 100 made of Teflon material the like, and then accommodated in the gas diffusion space.

In the gas analysis apparatus of the present disclosure, up to the process of mounting the battery to be analyzed 11 on the jig part 100 may be performed manually by the analyst, and subsequent processes may be performed automatically without any intervention of the analyst. Specifically, the process of loading the jig part 100 into the gas diffusion chamber 200, the process of diffusing gas into the gas diffusion space of the gas diffusion chamber 200, the process of delivering the diffused gas to the gas analysis unit, and the process of unloading the jig part 100 from the gas diffusion chamber 200 may be performed automatically without direct manipulation by the analyst.

As shown in FIG. 2, the jig part 100 may be made of Teflon material, a chemicalresistant material, as described above, and a battery accommodation groove 110 for mounting the battery to be analyzed 11 may be formed on an upper surface of the jig part 100. For example, the jig part 100 may be formed in a rectangular parallelepiped shape.

The gas analysis apparatus of the present disclosure may be capable of sequentially analyzing a plurality of batteries to be analyzed 11. A plurality of jig parts 100 may also be provided in order to correspond to the number of the plurality of batteries to be analyzed 11. Each of the plurality of batteries to be analyzed 11 may be mounted on each of the plurality of jig parts 100. At this time, the plurality of batteries to be analyzed 11 may may each be provided in different volumes or shapes. The plurality of jig parts 100 are all provided to have the same outer shape and specifications, but the size of the battery accommodation groove 110 is provided to correspond to the specifications of each of the batteries to be analyzed 11 which is mounted thereto, so that when the jig part 100 is accommodated in the gas diffusion space, a difference in volume of the space where the gas substantially diffuses may be made similar for each battery to be analyzed 11.

A gas discharge groove 130 may be formed on the upper surface of the jig part 100 so that the gas of the battery accommodation groove 110 may easily diffuse into the gas diffusion space. One end of the gas discharge groove 130 may be connected to the battery accommodation groove 110, and the other end may be located at the edge of the jig part 100. Accordingly, the gas of the battery accommodation groove 110 may escape from the side surface of the jig part 100 through the gas discharge groove 130 and diffuse into the gas diffusion space.

As shown in FIG. 3, the battery moving unit 500 may include a gripper part 510 for gripping the jig part 100, an elevation driving part 520 for linearly moving the gripper part 510 in a vertical direction, and a first driving part 530 for moving the gripper part 510 in a first direction perpendicular to the vertical direction.

When a direction perpendicular to the vertical direction and the first direction is referred to as a second direction, the gripper part 510 may include a pair of grip members 511 configured to grip the jig part 100 by contacting each of both side surfaces of the jig part 100 formed as a plane perpendicular to the second direction.

The pair of grip members 511 face each other, and the surfaces facing each other are formed as planes, so that they may grip the jig part 100 by being in close contact with both side surfaces of the jig part 100.

As shown in FIG. 4, a pair of gripper insertion groove 120 parts may be formed in the jig part 100, and each of the pair of gripper insertion groove 120 parts may be located on each of the both side surfaces of the jig part 100 formed as a plane perpendicular to the second direction. The gripper insertion groove 120 part may be formed at a location facing the grip member 511 when gripping the jig part 100 of the gripper part 510.

Each of the pair of gripper insertion groove 120 parts may include a first gripper insertion groove 121 formed in a long groove shape, and a second gripper insertion groove 122 formed to be spaced a predetermined distance apart from the first gripper insertion groove 121, respectively. Specifically, the first gripper insertion groove 121 may be provided in the shape of a long groove extending in the first direction. The second gripper insertion groove 122 may be formed on both side locations of the first gripper insertion groove 121 to be spaced a predetermined distance apart in the first direction. Therefore, two second gripper insertion grooves 122 may be allocated to each first gripper insertion groove 121. In other words, one jig part 100 may be provided with two first gripper insertion grooves 121 and four second gripper insertion grooves 122.

A first protrusion 511a to be inserted into the first gripper insertion groove 121 and a second protrusion 511b to be inserted into the second gripper insertion groove 122 may be formed on the pair of grip members 511. Specifically, the first protrusion 511a may be provided in a shape extending in the first direction to correspond to the shape of the first gripper insertion groove 121, and the second protrusion 511b may also be provided in a shape corresponding to the shape of the second gripper insertion groove 122. One first protrusion 511a and two second protrusions 511b may be formed on one grip member 511. As described above, since the jig part 100 is made of a material with low friction such as Teflon, when gripping the jig part 100 by the gripper part 510, the first protrusion 511a is inserted into the first gripper insertion groove 121, and the second protrusion 511b is inserted into the second gripper insertion groove 122, so that the jig part 100 may be stably gripped without shaking.

The gripper part 510 may further include a grip actuator 512 configured to provide driving force to adjust a gap between the pair of grip members 511, and a gripper body member 513 to which the grip actuator 512 and the pair of grip members 511 are coupled.

The grip actuator 512 may be an electric or pneumatic actuator.

The pair of grip members 511 may be fixed to the gripper body member 513 so that they are capable of sliding in the second direction.

The first driving part 530 may include a first moving member 531 configured to move linearly in the first direction, and a first guide member 532 configured to guide a movement of the first moving member 531, and the elevation driving part 520 may include an elevation guide member 522 coupled to the first moving member 531, and an elevation moving member 521 configured to move linearly in the vertical direction along the elevation guide member 522, wherein the gripper body member 513 may be fixed to the elevation moving member 521.

As shown in FIG. 5, the supply battery storage unit 300 may include a supply battery storage part 310 provided with the supply battery storage space inside and extending in the vertical direction, and a supply battery lifting part 320 configured to lift up the jig part 100 in the supply battery storage space, wherein a battery discharge port 311 may be formed at an upper end of the supply battery storage part 310, and a first gripper contact hole 312 may be formed on each of both side surfaces of the supply battery storage part 310 so that the pair of grip members 511 can directly contact each of the both side surfaces of the jig part 100 formed as a plane perpendicular to the second direction.

The supply battery storage part 310 may be provided in the shape of a square pillar extending in the vertical direction. In the supply battery storage space inside the supply battery storage part 310, the plurality of jig parts 100 may be stacked in the vertical direction and loaded. The supply battery lifting part 320 may support the bottom surface of the lowest jig part 100 among the plurality of jig parts 100 stacked in the vertical direction. When the gripper part 510 takes out one jig part 100 from the supply battery storage space through the battery discharge port 311, the supply battery lifting part 320 rises, and may lift up the height of the uppermost jig part 100 among the plurality of jig parts 100 located in the supply battery storage space to an appropriate location for the gripper part 510 to grip.

The first gripper contact holes 312 may be provided in pairs, and each of the pair of first gripper contact holes 312 may be formed in a shape that penetrates each of two planes perpendicular to the second direction of the supply battery storage part 310. The pair of first gripper contact holes 312 may be provided in a shape extending in the vertical direction, and the upper ends of the pair of first gripper contact holes 312 may be connected to the battery discharge port 311.

As shown in FIG. 6, the recovery battery storage unit 400 may include a recovery battery storage part 410 provided with the recovery battery storage space inside and extending in the vertical direction, and a recovery battery lowering part 420 configured to lower the jig part 100 in the recovery battery storage space, wherein a battery recovery port 411 may be formed at an upper end of the recovery battery storage part 410, and a second gripper contact hole 412 may be formed on each of both side surfaces of the recovery battery storage part 410 so that the pair of grip members 511 can directly contact each of the both side surfaces of the jig part 100 formed as a plane perpendicular to the second direction.

The recovery battery storage part 410 may be provided in the shape of a square pillar extending in the vertical direction. In the recovery battery storage space inside the recovery battery storage part 410, the plurality of jig parts 100 recovered from the gas diffusion chamber 200 may be stacked in the vertical direction and loaded. The recovery battery lowering part 420 may support the bottom surface of the lowest jig part 100 among the plurality of jig parts 100 stacked in the vertical direction. When the gripper part 510 loads the jig part 100 in the recovery battery storage space through the battery recovery port 411, the recovery battery lowering part 420 descends, and may lower the height of the uppermost jig part 100 among the plurality of jig parts 100 located in the recovery battery storage space to an appropriate location for the gripper part 510 to place.

The second gripper contact holes 412 may be provided in pairs, and each of the pair of second gripper contact holes 412 may be formed in a shape that penetrates each of two planes perpendicular to the second direction of the recovery battery storage part 410. The pair of second gripper contact holes 412 may be provided in a shape extending in the vertical direction, and the upper end of the pair of second gripper contact holes 412 may be connected to the battery recovery port 411.

The gas diffusion chamber 200 may be located to be spaced a predetermined distance apart from the supply battery storage unit 300 and the recovery battery storage unit 400 in the first direction. The gripper part 510 may move between the gas diffusion chamber 200 and the supply battery storage unit 300 or between the gas diffusion chamber 200 and the recovery battery storage unit 400 by the first driving part 530.

As shown in FIG. 7, the gas diffusion chamber 200 may include a chamber body part 210, wherein a jig part accommodation groove 211 is formed on an upper surface of the chamber body part 210 as the gas diffusion space, and a chamber cover part 220 configured to cover an upper surface of the chamber body part 210 to make the gas diffusion space a closed space, and the chamber cover part 220 may be configured to open or close the gas diffusion space by sliding in a second direction perpendicular to the vertical direction and the first direction.

A sealing member insertion groove 212 may be formed on the upper surface of the chamber body part 210 around the jig part accommodation groove 211. Specifically, the sealing member insertion groove 212 may be provided in a closed loop shape so that a sealing member such as an O-ring may be inserted, and the jig part accommodation groove 211 may be located inside the inner circumference of the sealing member insertion groove 212.

The upper surface of the chamber body part 210 and the bottom surface of the chamber cover part may be formed as planes to be completely adhered to each other.

In one embodiment, the battery moving unit may further include a second driving part (not shown) to locate the center of the gripper body member 513 of the gripper part 510 together with one of the center of the jig part accommodation groove 211, the center of the discharge port 311 of the supply battery storage part 310 and the center of the recovery port 411 of the recovery battery storage part 410 together on an imaginary plane parallel to the first direction and the vertical direction. In other words, the second driving part may cause the center of the gripper body member 513 of the gripper part 510 to be in the same position as one of the center of the jig part accommodation groove 211, the center of the discharge port 311 of the supply battery storage part 310 and the center of the recovery port 411 of the recovery battery storage part 410 in the second direction. The second driving part may be mounted on the first moving member 531 or the elevation moving member 521.

In another embodiment, the center of the discharge port 311 of the supply battery storage part 310, the center of the recovery port 411 of the recovery battery storage part 410, and the center of the jig part accommodation groove 211 may be located on an imaginary plane parallel to the first direction and the vertical direction. In other words, the center of the discharge port 311 of the supply battery storage part 310, the center of the recovery port 411 of the recovery battery storage part 410, and the center of the jig part accommodation groove 211 may be located at the same position in the second direction. Accordingly, the distance between the supply battery storage unit 300 and the gas diffusion chamber 200 may be formed to be farther or closer than the distance between the recovery battery storage unit 400 and the gas diffusion chamber 200. In other words, on an imaginary straight line parallel to the first direction, they may be arranged in the order of [gas diffusion chamber 200] - [supply battery storage unit 300] - [recovery battery storage unit 400] or arranged in the order of [gas diffusion chamber 200] - [recovery battery storage unit 400] - [supply battery storage unit 300]. In this case, the second driving part may be omitted.

A punching part 221 for perforating the battery to be analyzed 11 may be provided on an upper surface of the chamber cover part 220, wherein a punching needle of the punching part 221 may be configured to perforate the battery to be analyzed 11 by being inserted into the gas diffusion space through a penetration hole of the chamber cover part 220.

The punching part 221 may move the punching needle in the vertical direction through an electric motor or actuator.

The gas delivery flow path 290 may be connected to the chamber body part 210. The gas delivery flow path 290 may include a tube, hose, pipe, etc., through which gas may move.

A manifold may be provided in the gas delivery flow path 290, and the gas delivered from the gas diffusion space to the gas analysis unit through the gas delivery flow path 290 may be injected into the gas analysis unit after waiting in the manifold for a predetermined period of time.

A gas collection container for storing the gas diffused in the gas diffusion space, a dilution chamber for diluting the gas stored in the gas collection container, and a vacuum pump may be connected to the manifold.

Although embodiments according to the present disclosure have been described above, they are merely illustrative and those skilled in the art will understand that various modifications and embodiments of equivalent range are possible therefrom. Therefore, the true technical protection scope of the present disclosure should be defined by the following claims.

<Explanation of Symbols> 11...Battery to be analyzed, 100...Jig part, 110...Battery accommodation groove, 120...Gripper insertion groove, 121...First gripper insertion groove, 122...Second gripper insertion groove, 130...Gas discharge groove, 200...Gas diffusion chamber, 210...Chamber body part, 211...Jig part accommodation groove, 212...Sealing member insertion groove, 220...Chamber cover part, 221...Punching part, 290...Gas delivery flow path, 300...Supply battery storage unit, 310...Supply battery storage part, 311...Battery discharge port, 312...First gripper contact hole, 320...Supply battery lifting part, 400...Recovery battery storage unit, 410...Recovery battery storage part, 411...Battery recovery port, 412...Second gripper contact hole, 420...Recovery battery lowering part, 500...Battery moving unit, 510...Gripper part, 511...Grip member, 511a...First protrusion, 511b...Second protrusion, 512...Grip actuator, 513...Gripper body member, 520...Elevation driving part, 521...Elevation moving member, 522...Elevation guide member, 530...First driving part, 531...First moving member, 532...First guide member

### Industrial Applicability

A gas analysis apparatus of the present disclosure is an automated system which may be capable of collecting and analyzing secondary battery generated gas by minimizing the intervention of the analyst.

The gas analysis apparatus of the present disclosure may be capable of independently collecting and analyzing gases for a plurality of secondary batteries with only the initial settings of the analyst.

The gas analysis apparatus of the present disclosure may be capable of continuous analysis even if the analyst is not waiting near the gas analysis apparatus.

## Claims

1. A gas analysis apparatus comprising:
a jig part on which a battery to be analyzed is mounted;
a gas diffusion chamber provided with a gas diffusion space in which the jig part is accommodated inside;
a supply battery storage unit provided with a supply battery storage space in which the jig part for supplying to the gas diffusion space is stored;
a recovery battery storage unit provided with a recovery battery storage space in which the jig part recovered from the gas diffusion space is stored;
a battery moving unit configured to move the jig part from the battery supply part to the gas diffusion space or move the jig part accommodated in the gas diffusion space to the battery recovery part;
a gas analysis unit configured to analyze gas delivered from the gas diffusion space; and
a gas delivery flow path configured to connect the gas diffusion space and the gas analysis unit.

2. The gas analysis apparatus of claim 1, wherein a battery accommodation groove for mounting the battery to be analyzed is formed on an upper surface of the jig part.

3. The gas analysis apparatus of claim 1, wherein the battery moving unit comprises:
a gripper part for gripping the jig part;
an elevation driving part for linearly moving the gripper part in a vertical direction; and
a first driving part for moving the gripper part in a first direction perpendicular to the vertical direction.

4. The gas analysis apparatus of claim 3, wherein, when a direction perpendicular to the vertical direction and the first direction is referred to as a second direction,
the gripper part comprises a pair of grip members configured to grip the jig part by contacting each of both side surfaces of the jig part formed as a plane perpendicular to the second direction.

5. The gas analysis apparatus of claim 4, wherein a pair of gripper insertion groove parts are formed in the jig part, and
each of the pair of gripper insertion groove parts is located on each of the both side surfaces of the jig part formed as the plane perpendicular to the second direction.

6. The gas analysis apparatus of claim 5, wherein each of the pair of gripper insertion groove parts comprises:
a first gripper insertion groove formed in a long groove shape; and
a second gripper insertion groove formed to be spaced a predetermined distance apart from the first gripper insertion groove.

7. The gas analysis apparatus of claim 6, wherein a first protrusion to be inserted into the first gripper insertion groove and a second protrusion to be inserted into the second gripper insertion groove are formed on the pair of grip members.

8. The gas analysis apparatus of claim 4, wherein the gripper part further comprises a grip actuator configured to provide driving force to adjust a gap between the pair of grip members, and a gripper body member to which the grip actuator and the pair of grip members are coupled,
the first driving part comprises a first moving member configured to move linearly in the first direction, and a first guide member configured to guide a movement of the first moving member,
the elevation driving part comprises an elevation guide member coupled to the first moving member, and an elevation moving member configured to move linearly in the vertical direction along the elevation guide member, and
the gripper body member is fixed to the elevation moving member.

9. The gas analysis apparatus of claim 4, wherein the supply battery storage unit comprises:
a supply battery storage part provided with the supply battery storage space inside and extending in the vertical direction; and
a supply battery lifting part configured to lift up the jig part in the supply battery storage space,
a battery discharge port is formed at an upper end of the supply battery storage part, and
a first gripper contact hole is formed on each of both side surfaces of the supply battery storage part so that the pair of grip members can directly contact each of the both side surfaces of the jig part formed as the plane perpendicular to the second direction.

10. The gas analysis apparatus of claim 4, wherein the recovery battery storage unit comprises:
a recovery battery storage part provided with the recovery battery storage space inside and extending in the vertical direction; and
a recovery battery lowering part configured to lower the jig part in the recovery battery storage space,
a battery recovery port is formed at an upper end of the recovery battery storage part, and
a second gripper contact hole is formed on each of both side surfaces of the recovery battery storage part so that the pair of grip members can directly contact each of the both side surfaces of the jig part formed as the plane perpendicular to the second direction.

11. The gas analysis apparatus of claim 3, wherein the gas diffusion chamber is located to be spaced a predetermined distance apart from the supply battery storage unit and the recovery battery storage unit in the first direction, and
wherein the gas diffusion chamber comprises:
a chamber body part, wherein a jig part accommodation groove is formed on an upper surface of the chamber body part as the gas diffusion space; and
a chamber cover part configured to cover the upper surface of the chamber body part to make the gas diffusion space a closed space, and
the chamber cover part is configured to open or close the gas diffusion space by sliding in a second direction perpendicular to the vertical direction and the first direction.

12. The gas analysis apparatus of claim 11, wherein a punching part for perforating the battery to be analyzed is provided on an upper surface of the chamber cover part, and
a punching needle of the punching part is configured to perforate the battery to be analyzed by being inserted into the gas diffusion space through a penetration hole of the chamber cover part.

13. The gas analysis apparatus of claim 11, wherein the gas delivery flow path is connected to the chamber body part.

14. The gas analysis apparatus of claim 13, wherein a manifold is provided in the gas delivery flow path, and
the gas delivered from the gas diffusion space to the gas analysis unit through the gas delivery flow path is injected into the gas analysis unit after waiting in the manifold for a predetermined period of time.
